# EUROPEAN PATENT APPLICATION

(11) **EP 4 368 187 A1**
(43) Date of publication of application: **15.05.2024**
(21) Application number: 22837760.2
(22) Date of filing: 08.07.2022
(51) Int. Cl.: A61K 31/70, A61K 31/7016, A61K 31/7088, A61K 31/712, A61K 31/7125, A61K 47/26, A61P 13/02, A61P 13/12, C12N 15/113

(54) **PRECIPITATION SUPPRESSING AGENT**

(30) Priority: 08.07.2021 JP 2021113562
(71) Applicant: Nippon Shinyaku Co., Ltd., Kyoto-shi Kyoto 601-8550 (JP)
(72) Inventor: ENYA, Yukiko, Tsukuba-shi, Ibaraki 305-0003 (JP); NUMAKURA, Yuki, Kyoto-shi, Kyoto 601-8550 (JP); OKAMOTO, Kentaro, Kyoto-shi, Kyoto 601-8550 (JP); AGATA, Kazuki, Kyoto-shi, Kyoto 601-8550 (JP)
(74) Representative: J A Kemp LLP
(86) International application number: PCT/JP2022/027105
(87) International publication number: WO 2023/282346

(57) **Abstract**

In one embodiment, the object of the present invention is to provide a precipitation suppressing agent for an antisense oligomer in urine of a subject to whom a pharmaceutical composition comprising an antisense oligomer has been administered, and a method for suppressing precipitation of an antisense oligomer in urine, in a subject to whom the pharmaceutical composition has been administered. In one embodiment, the present invention relates to a precipitation suppressing agent for an antisense oligomer in urine of a subject to whom a pharmaceutical composition comprising an antisense oligomer has been administered, wherein the precipitation suppressing agent comprises a sugar other than glucose and is used in an amount such that the concentration of the sugar in the pharmaceutical composition is 0.5 mg/mL to 3000 mg/mL.

## Description

### Technical Field

The present invention relates to a precipitation suppressing agent comprising a sugar for an antisense oligomer in urine of a subject to whom a pharmaceutical composition comprising an antisense oligomer has been administered, a method for suppressing precipitation of an antisense oligomer in urine, in a subject to whom the pharmaceutical composition has been administered, a pharmaceutical composition comprising the precipitation suppressing agent, and the like.

### Background Art

Antisense oligomers are nucleic acids that sequencespecifically hybridize to target mRNAs and pre-mRNAs. The antisense oligomers exert their actions by degradation of mRNAs and pre-mRNAs, exon-skipping, exoninclusion, translation inhibition and the like, and thus have been used as therapeutic agents for some diseases. For example, Japanese Patent Laid-Open No. 2015-91229 (Patent Literature 1) discloses an antisense nucleic acid which can treat Fukuyama muscular dystrophy by normalizing aberrant splicing of a fukutin gene having an insertion mutation of SVA retrotransposon.

However, with respect to the antisense oligomers it has been known that, for example, morpholino oligomers are accumulated in the kidney (Non Patent Literature 1, Figure 2), administration of morpholino oligomers causes nephrotoxicity to be expressed (Non Patent Literature 2, Figure 3, Table 5), and the administration of morpholino oligomers induces basophilic substances in the kidney tubule (Non Patent Literature 3, Figure 1). A method for avoiding the appearance of the nephrotoxicity and the basophilic substances in the renal tubule has not been established. On the other hand, it has been known the following: for oligonucleotides that have greater toxicity in the aggregate form than in monomeric form, heating with use of chemical species such as mannitol that disrupt aggregates can suppress multimerization in formulations of therapeutic oligonucleotides (Patent Literature 2); and saccharides and sugar alcohols in liquid formulations suppress aggregation of oligonucleotides (Patent Literature 3).

### Citation List

### Patent Literature

Patent Literature 1: Japanese Patent Laid-Open No. 2015-91229
Patent Literature 2: International Publication No. 2002/036767
Patent Literature 3: International Publication No. 2010/009038

### Non Patent Literature

Non Patent Literature 1: Heemskerk HA, de Winter CL, de Kimpe SJ, van Kuik-Romeijn P, Heuvelmans N, Platenburg GJ, van Ommen GJ, van Deutekom JC, Aartsma-Rus A. In vivo comparison of 2'-O-methyl phosphorothioate and morpholino antisense oligonucleotides for Duchenne muscular dystrophy exon skipping. J Gene Med. 2009 Mar; 11(3):257-66. doi: 10.1002/jgm.1288.
Non Patent Literature 2: Sazani P, Ness KP, Weller DL, Poage D, Nelson K, Shrewsbury AS. Chemical and mechanistic toxicology evaluation of exon skipping phosphorodiamidate morpholino oligomers in mdx mice. Int J Toxicol. 2011 May; 30(3):322-33. doi: 10.1177/1091581811403504. Epub 2011 May 3.
Non Patent Literature 3: Carver MP, Charleston JS, Shanks C, Zhang J, Mense M, Sharma AK, Kaur H, Sazani P. Toxicological Characterization of Exon Skipping Phosphorodiamidate Morpholino Oligomers (PMOs) in Nonhuman Primates. J Neuromuscul Dis. 2016 Aug 30; 3(3):381-393. doi: 10.3233/JND-160157.

### Summary of Invention

### Technical Problem

In such a circumstance, it is desirable to provide an improved pharmaceutical composition comprising antisense oligomers.

### Solution to Problem

That is, the present invention provides a precipitation suppressing agent comprising a sugar other than glucose such as those described below for an antisense oligomer in urine of a subject to whom a pharmaceutical composition comprising an antisense oligomer has been administered, a method for suppressing precipitation of an antisense oligomer in urine in a subject to whom the pharmaceutical composition has been administered, a pharmaceutical composition comprising the precipitation suppressing agent, and the like.
(1) A precipitation suppressing agent for an antisense oligomer in urine of a subject to whom a pharmaceutical composition comprising an antisense oligomer has been administered, wherein the precipitation suppressing agent comprises a sugar other than glucose and is used in an amount such that the concentration of the sugar in the pharmaceutical composition is 0.5 mg/mL to 3000 mg/mL.
(2) The precipitation suppressing agent according to (1), wherein the precipitation suppressing agent is used in an amount such that the concentration of the sugar in the pharmaceutical composition is 2.6 mg/mL to 1694.1 mg/mL.
(3) The precipitation suppressing agent according to (1) or (2), wherein the concentration of the antisense oligomer in the pharmaceutical composition is 0.05 mg/mL to 2000 mg/mL.
(4-1) A precipitation suppressing agent for an antisense oligomer in urine of a subject to whom a pharmaceutical composition comprising an antisense oligomer has been administered, wherein the precipitation suppressing agent comprises a sugar other than glucose and is used in an amount such that the weight ratio of the sugar is 0.1 to 100 per 1 of the antisense oligomer.
(4-2) The precipitation suppressing agent according to (4-1), wherein the pharmaceutical composition and the precipitation suppressing agent are administered separately.
(5) The precipitation suppressing agent according to (4-1) or (4-2), wherein the precipitation suppressing agent is used in an amount such that the weight ratio of the sugar is 1 to 53 per 1 of the antisense oligomer.
(6) The precipitation suppressing agent according to any of (1) to (5), wherein the sugar is a disaccharide.
(7) The precipitation suppressing agent according to any of (1) to (6), wherein the sugar is sucrose.
(8) The precipitation suppressing agent according to any of (1) to (6), wherein the sugar is trehalose.
(9) The precipitation suppressing agent according to any of (1) to (8), wherein the antisense oligomer is a morpholino oligomer.
(10) The precipitation suppressing agent according to any of (1) to (9), wherein the antisense oligomer is a phosphorodiamidate morpholino oligomer.
(11) The precipitation suppressing agent according to any of (1) to (10), wherein the 5'-end of the antisense oligomer is any group represented by the following chemical formulae (1) and (2): 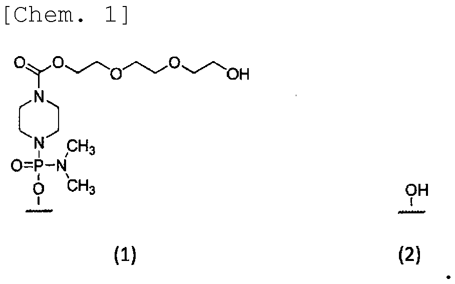
(12) The precipitation suppressing agent according to any of (1) to (11), wherein the antisense oligomer comprises four consecutive purine bases in its base sequence.
(13) The precipitation suppressing agent according to (12), wherein at least two of the four consecutive purine bases are guanine.
(14) The precipitation suppressing agent according to any of (1) to (13), wherein the antisense oligomer comprises a base sequence selected from the group consisting of SEQ ID NOs: 1 to 12.
(15-1) A method for suppressing precipitation of an antisense oligomer in urine, in a subject to whom a pharmaceutical composition comprising an antisense oligomer has been administered, comprising adding a sugar other than glucose in an amount such that the concentration of the sugar in the pharmaceutical composition is 0.5 mg/mL to 3000 mg/mL.
(15-2) The method according to (15-1), wherein the sugar is added in an amount such that the concentration of the sugar in the pharmaceutical composition is 2.6 mg/mL to 1694.1 mg/mL.
(15-3) The method according to (15-1) or (15-2), wherein the concentration of the antisense oligomer in the pharmaceutical composition is 0.05 mg/mL to 2000 mg/mL.
(16-1) A method for suppressing precipitation of an antisense oligomer in urine, in a subject to whom an antisense oligomer has been administered, comprising administering a sugar other than glucose to the subject, wherein the sugar is administered in an amount such that the weight ratio of the sugar is 0.1 to 100 per 1 of the antisense oligomer.
(16-2) The method according to (16-1), wherein the antisense oligomer and the sugar are administered separately.
(16-3) The method according to (16-1) or (16-2), wherein the sugar is used in an amount such that the weight ratio of the sugar is 1 to 53 per 1 of the antisense oligomer.
(17-1) A pharmaceutical composition comprising an antisense oligomer, wherein the pharmaceutical composition comprises a precipitation suppressing agent at the concentration of 0.5 mg/mL to 3000 mg/mL for an antisense oligomer in urine of a subject to whom the pharmaceutical composition has been administered, the precipitation suppressing agent comprising a sugar other than glucose.
(17-2) The pharmaceutical composition according to (17-1), wherein the pharmaceutical composition comprises the precipitation suppressing agent comprising a sugar at the concentration of 2.6 mg/mL to 1694.1 mg/mL.
(17-3) The pharmaceutical composition according to (17-1) or (17-2), wherein the concentration of the antisense oligomer in the pharmaceutical composition is 0.05 mg/mL to 2000 mg/mL.
(18) The method or pharmaceutical composition according to any of (15-1) to (17-3), wherein the sugar is a disaccharide.
(19) The method or pharmaceutical composition according to any of (15-1) to (18), wherein the sugar is sucrose.
(20) The method or pharmaceutical composition according to any of (15-1) to (18), wherein the sugar is trehalose.
(21) The method or pharmaceutical composition according to any of (15-1) to (20), wherein the antisense oligomer is a morpholino oligomer.
(22) The method or pharmaceutical composition according to any of (15-1) to (21), wherein the antisense oligomer is a phosphorodiamidate morpholino oligomer.
(23) The method or pharmaceutical composition according to any of (15-1) to (22), wherein the 5'-end of the antisense oligomer is any group represented by the following chemical formulae (1) and (2): 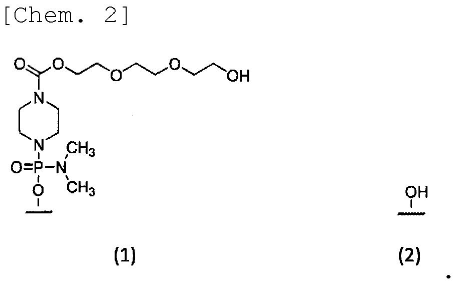
(24) The method or pharmaceutical composition according to any of (15-1) to (23), wherein the antisense oligomer comprises four consecutive purine bases in its base sequence.
(25) The method or pharmaceutical composition according to (24), wherein at least two of the four consecutive purine bases are guanine.
(26) The method or pharmaceutical composition according to any of (15-1) to (25), wherein the antisense oligomer comprises a base sequence selected from the group consisting of SEQ ID NOs: 1 to 12.

### Advantageous Effects of Invention

The present invention provides a precipitation suppressing agent for an antisense oligomer in urine of a subject to whom a pharmaceutical composition comprising an antisense oligomer has been administered, and a method for suppressing precipitation of an antisense oligomer in urine, in a subject to whom the pharmaceutical composition has been administered.

### Brief Description of Drawings

[Figure 1] Figure 1 shows absorbance measurement values at 620 nm under the conditions shown in Table 4.
[Figure 2] Figure 2 shows absorbance measurement values at 620 nm under the conditions shown in Table 5.
[Figure 3] Figure 3 shows absorbance measurement values at 620 nm under the conditions shown in Table 6.
[Figure 4] Figure 4 shows absorbance measurement values at 620 nm under the conditions shown in Table 7.
[Figure 5] Figure 5 shows absorbance measurement values at 620 nm under the conditions shown in Table 8.
[Figure 6] Figure 6 shows absorbance measurement values at 620 nm under the conditions shown in Table 9.
[Figure 7] Figure 7 shows absorbance measurement values at 620 nm under the conditions shown in Table 10.
[Figure 8] Figure 8 shows absorbance measurement values at 620 nm under the conditions shown in Table 11.
[Figure 9] Figure 9 shows a hematoxylin-eosin staining image of a kidney when a PMO No. 8 dosing solution (50 mg/mL of PMO No. 8) is administered at 10 mL/kg.
[Figure 10] Figure 10 shows a hematoxylin-eosin staining image of a kidney when PMO No. 8 dosing solution(50 mg/mL of PMO No. 8, and 20 mg/mL of sucrose) is administered at 10 mL/kg.
[Figure 11] Figure 11 shows a hematoxylin-eosin staining image of a kidney when a PMO No. 8 dosing solution (50 mg/mL of PMO No. 8, and 50 mg/mL of sucrose) is administered at 10 mL/kg.
[Figure 12] Figure 12 shows a hematoxylin-eosin staining image of a kidney when a PMO No. 8 dosing solution (50 mg/mL of PMO No. 8, and 100 mg/mL of sucrose) is administered at 10 mL/kg.
[Figure 13] Figure 13 shows absorbance measurement values at 620 nm under the conditions shown in Table 15.
[Figure 14] Figure 14 shows absorbance measurement values at 620 nm under the conditions shown in Table 16.
[Figure 15] Figure 15 shows absorbance measurement values at 620 nm under the conditions shown in Table 17.
[Figure 16] Figure 16 shows absorbance measurement values at 620 nm under the conditions shown in Table 18.
[Figure 17] Figure 17 shows absorbance measurement values at 620 nm under the conditions shown in Table 19.
[Figure 18] Figure 18 shows absorbance measurement values at 620 nm under the conditions shown in Table 20.
[Figure 19] Figure 19 shows absorbance measurement values at 620 nm under the conditions shown in Table 21.

### Description of Embodiments

In one embodiment, the present invention relates to a precipitation suppressing agent for an antisense oligomer in urine of a subject to whom a pharmaceutical composition comprising an antisense oligomer has been administered. The term "precipitation suppressing agent" as used herein means an agent for suppressing precipitation of an antisense oligomer in urine of a subject to whom a pharmaceutical composition comprising an antisense oligomer has been administered. If suppression of precipitation results in the reduced accumulation of the antisense oligomer in the kidney in vivo, it is expected to lead to reduced nephrotoxicity. The term nephrotoxicity means occurrence of tissue damage or a decrease in kidney function due to higher accumulation of the administered antisense oligomers in the kidney. Also, If precipitation in body fluid such as blood is suppressed in vivo, it is expected to cause the concentration of the antisense oligomers in the body fluid to be homogeneous, leading to stabilization of agent efficacy and/or reduced toxicity.

The presence or absence of the precipitation suppressing effects can be determined by adding the antisense oligomer and the precipitation suppressing agent in a solvent simulating urine and examining as described in Examples whether or not the precipitation suppressing agent reduces an increase in absorbance caused by precipitation of the antisense oligomer. For example, after the antisense oligomer and the precipitation suppressing agent are added to the solvent (e.g., 5 minutes later, 10 minutes later, 15 minutes later, 20 minutes later, 25 minutes later, 30 minutes later, 40 minutes later, 50 minutes later, 60 minutes later, 70 minutes later, 80 minutes later, or 90 minutes later), the absorbance can be determined under the conditions of 37°C. At this time, compared to a case of adding no precipitation suppressing agent, it can be determined to be effective in suppressing precipitation when a time until the precipitation is observed (until the increased absorbance at 620 nm is observed) or the absorbance at 620 nm are reduced, for example, by 5% or more, 100 or more, 20% or more, 30% or more, 40% or more, or 50% or more in the case of adding the precipitation suppressing agent. Examples of the solvent simulating urine that can be used include an aqueous solution comprising KCl at 20 nmol/mL to 9000 nmol/mL, 50 nmol/mL to 3600 nmol/mL, or 100 nmol/mL to 1800 nmol/mL, and NaCl at 15 nmol/mL to 2400 nmol/mL, 40 nmol/mL to 900 nmol/mL, or 77 nmol/mL to 462 nmol/mL.

Since it can be assumed from the results of Examples of the present application that there can be a linear relationship between the amount of the sugar administered to the subject and the urinary concentration, the sugar concentration (hereinafter, also referred to as "urinary concentration") in the solvent simulating urine can be converted into sugar concentration in a formulation as follows. For example, based on the results of administering 500 mg/kg of mannitol to cynomolgus monkeys, the urinary concentration can be converted into the amount of the sugar administered by using the following formulas, and further, the amount of the sugar administered can be converted into concentrations in the formulations at the time of administration (at the time of use) according to the body weight and the amount of dosing solution.

Amount of sugar administered (mg/kg) = urinary concentration (mg/mL)/estimated urinary concentration in cynomolgus monkey (127.5 (mg/mL)) × amount administered to cynomolgus monkey (500 (mg/kg)) D-mannitol concentration in formulation (mg/mL) = amount of D-mannitol administered (mg/kg) × body weight (kg)/amount of dosing solution (mL)

The body weight and the amount of dosing solution can be assumed to be, for example, 3 to 80 kg and 50 to 200 mL, respectively.

Similarly, the concentration of antisense oligomers in the solvent simulating urine can be converted into concentration of antisense oligomers in formulation as described in Examples.

In one embodiment, the precipitation suppressing agent of the present invention may consist of or comprise a sugar (except glucose). The sugar may be a sugar of two or more saccharides or may be a disaccharide. Examples of the sugar include, but not limited to, disaccharides such as sucrose, lactose, lactulose, trehalose, maltose, and isomaltose; trisaccharides such as raffinose, melezitose, and maltotriose; and monosaccharides such as galactose, mannose, fructose, ribose, xylose, arabinose, and lyxose. In one embodiment, the sugar is or comprises sucrose. In another embodiment, the sugar is or comprises trehalose.

When comprising an ingredient other than the sugar, the precipitation suppressing agent of the present invention can be formulated by appropriately blending a pharmaceutically acceptable carrier or additive (and optionally the precipitation suppressing agent of the present invention) to the sugar. Specifically, it can be formulated into oral agents such as tablets, coated tablets, pills, powders, granules, capsules, liquids, suspensions, and emulsions; and parenteral agents such as injectables, infusions, suppositories, ointments and patches. The parenteral agents are preferable. The injectables may be lyophilized preparations. The blending proportion of the carrier or additive can be appropriately set according to the range usually employed in the pharmaceutical field. Examples of the carriers or additives to be blended include, but not particularly limited, various carriers such as water, physiological saline, other aqueous solvents, and aqueous or oily bases, and various additives such as excipients, binders, pH adjusters, disintegrants, absorption promoters, lubricants, colorants, corrigents, and flavors.

The additives miscible with tablets, capsules or the like are used that include, for example, binders such as gelatin, corn starch, tragacanth, or gum arabic, excipients such as crystalline cellulose, swelling agents such as corn starch, gelatin, or alginic acid, lubricants such as magnesium stearate, sweetening agents such as sucrose, lactose, or saccharin, flavoring agents such as peppermint, Gaultheria adenothrix oil, or cherries. When the formulated dosage unit form is a capsule, a liquid carrier such as oil/fat can be further comprised in the materials of the above types. A sterile composition for injection can be prepared according to the normal pharmaceutical practice (e.g., dissolution or suspension of active ingredients into solvents such as water for injection, or natural vegetable oil). As the aqueous solutions for injection, isotonic solutions (e.g., sodium chloride) comprising physiological saline, glucose, or other aid agents are used and can be combined with an adequate solubilizer such as alcohol (e.g., ethanol), polyalcohol (e.g., propylene glycol or polyethylene glycol), and a nonionic surfactant (e.g., polysorbate 80 (TM) or HCO-50). As the oily solutions, for example, sesame oil or soybean oil is used and can be combined with a solubilizer, such as benzyl benzoate or benzyl alcohol. In addition, buffers (e.g., phosphate buffer solution or sodium acetate buffer solution), soothing agents (e.g., benzalkonium chloride or procaine hydrochloride), stabilizers (e.g., human serum albumin or polyethylene glycol), preservatives (e.g., benzyl alcohol or phenol), or antioxidants may be blended therewith. Further, it can be lyophilized preparations.

As used herein, the antisense oligomer may be any of an oligonucleotide, a morpholino oligomer, or a peptide nucleic acid (PNA) oligomer (hereinafter, also referred to as "antisense oligonucleotide described herein", "antisense morpholino oligomer described herein", "antisense peptide nucleic acid oligomer described herein", respectively).

The antisense oligonucleotide is an antisense oligomer whose constituent unit is a nucleotide, and the nucleotide may be any of a ribonucleotide, a deoxyribonucleotide, or a modified nucleotide.

The modified nucleotide refers to those in which all or part of nucleobase, sugar moiety and phosphate-binding moiety that are constituents of ribonucleotides or deoxyribonucleotides are modified.

Examples of the nucleobases can include adenine, guanine, hypoxanthine, cytosine, thymine, uracil, or modified bases thereof. Examples of the modified bases include, but not limited to, pseudouracil, 3-methyluracil, dihydrouracil, 5-alkynecytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 2-thiouracil, 4-thiouracil, 4-acetylcytosine, 5-(carboxyhydroxymethyl)uracil, 5-carboxymethylaminomethyl-2-thiouracil, 5-carboxymethylaminomethyl uracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, and xanthine.

Examples of the modification of the sugar moiety can include modification at the 2' position of ribose, and modification of other parts of the sugar. Examples of the modification at the 2' position of ribose can include modification wherein -OH group at the 2' position of ribose is substituted with -OR, -R, -R'OR, -SH, -SR, - NH₂, -NHR, -NR₂, -N₃, -CN, -F, -Cl, -Br, and -I. Here, R represents alkyl or aryl. R' represents alkylene.

Examples of the modification of other parts of the sugar include, but not limited to, those in which O at the 4' position of ribose or deoxyribose is substituted with S, and the 2' and 4' positions of the sugar are cross-linked, for example, a locked nucleic acid (LNA) or 2'-O,4'-C-ethylene-bridged nucleic acid (ENA).

Examples of the modification of phosphate-binding moiety can include modifications wherein the phosphodiester bond is substituted with a phosphorothioate bond, a phosphorodithioate bond, an alkylphosphonate bond, a phosphoramidate bond, and a boranophosphate bond (see, e.g., Enya et al: Bioorganic & Medicinal Chemistry, 2008, 18, 9154-9160) (see, e.g., Japanese Re-Publication of International Patent Application Nos. 2006/129594 and 2006/038608).

As used herein, the alkyl is preferably a linear or branched alkyl having 1 to 6 carbon atoms. Specific examples thereof include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may be substituted, examples of the substituent therefor can include halogen, alkoxy, cyano, and nitro, and 1 to 3 positions may be substituted with these substituents.

As used herein, the cycloalkyl is preferably those having 3 to 12 carbon atoms. Specific examples thereof include cyclopropyl, cyclobutyl, cyclopentyl, cyclohexyl, cycloheptyl, cyclooctyl, cyclodecyl, and cyclododecyl.

As used herein, examples of the halogen can include fluorine, chlorine, bromine, and iodine.

As used herein, examples of the alkoxy include a linear or branched alkoxy having 1 to 6 carbon atoms, and for example, methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Alkoxy having 1 to 3 carbon atoms is preferable among others.

As used herein, the aryl is preferably those having 6 to 10 carbon atoms. Specific examples thereof can include phenyl, α-naphthyl, and β-naphthyl. Phenyl is preferable among others. The aryl may be substituted, examples of the substituent therefor can include alkyl, halogen, alkoxy, cyano, and nitro, and the aryl may be substituted with 1 to 3 of these substituents.

As used herein, the alkylene is preferably a linear or branched alkylene having 1 to 6 carbon atoms. Specific examples thereof can include methylene, ethylene, trimethylene, tetramethylene, pentamethylene, hexamethylene, 2-(ethyl)trimethylene, and 1-(methyl)tetramethylene.

As used herein, examples of acyl can include a linear or branched alkanoyl or aroyl. Examples of the alkanoyl can include formyl, acetyl, 2-methylacetyl, 2,2-dimethylacetyl, propionyl, butyryl, isobutyryl, pentanoyl, 2,2-dimethylpropionyl, and hexanoyl. Examples of the aroyl can include benzoyl, toluoyl, and naphthoyl. The aroyl may be substituted at a substitutable position, or may be substituted with alkyl.

The antisense oligonucleotide described herein can be easily synthesized with various automated synthesizers (e.g., AKTA oligopilot plus 10 / 100 (GE Healthcare)). Alternatively, it can be made by entrusting any third party institution (e.g., Promega Corporation or Takara Bio Inc.).

The antisense morpholino oligomer described herein is an antisense oligomer whose constituent unit is a group represented by the following general formula: 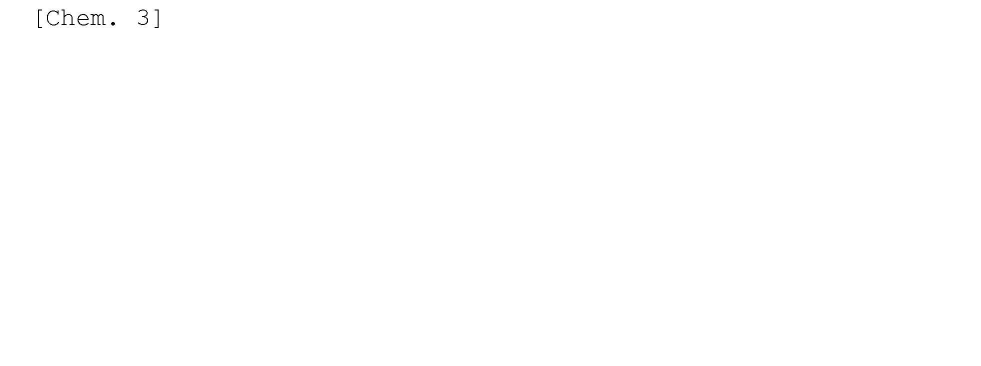 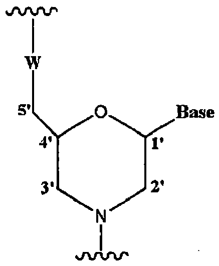
wherein, Base represents a nucleobase; and
   W represents a group represented by any of the following formulae: 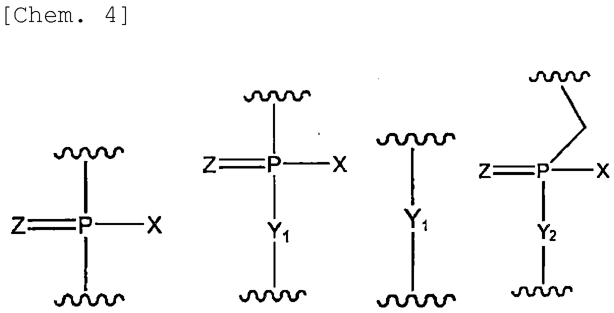
wherein, X represents -CH₂R¹, -O-CH₂R¹, -S-CH₂R¹, -NR²R³, or F;
   R¹ represents H, or alkyl;
   R² and R³ are the same or different, and represent H, alkyl, cycloalkyl, or aryl;
   Y₁ represents O, S, CH₂, or NR¹;
   Y₂ represents O, S, or NR¹; and
   Z represents O or S.

As used herein, the morpholino oligomer is preferably an oligomer whose constituent unit is a group represented by the following formula (phosphorodiamidate morpholino oligomer, hereinafter referred to as "PMO"): 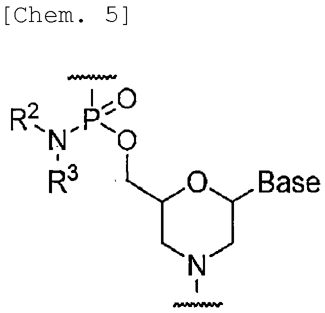 wherein, Base, R² and R³ are as defined above.

The morpholino oligomer can be produced according to the methods described in International Publication Nos. 1991/009033, or 2009/064471, for example. Particularly, PMO can be produced according to the methods described in International Publication Nos. 2009/064471, or 2013/100190.

The antisense peptide nucleic acid oligomer described herein is an antisense oligomer whose constituent unit is a group represented by the following general formula: 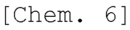 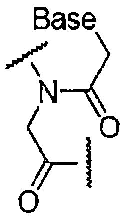 wherein, Base is as defined above.

The peptide nucleic acid oligomer can be produced, for example, according to the following references:
1) P. E. Nielsen, M. Egholm, R. H. Berg, O. Buchardt, Science, 254, 1497 (1991);
2) M. Egholm, O. Buchardt, P. E. Nielsen, R. H. Berg, JACS, 114, 1895 (1992);
3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994);
4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995); and
5) T. Koch, H. F. Hansen, P. Andersen, T. Larsen, H. G. Batz, K. Otteson, H. Orum, J. Pept. Res., 49, 80 (1997).

The antisense oligomer described herein may be in the form of a pharmaceutically acceptable salt, in the form of a hydrate, or in the form of a hydrate of a pharmaceutically acceptable salt thereof.

Examples of the pharmaceutically acceptable salt of the antisense oligomer described herein include alkali metal salts such as a sodium salt, a potassium salt, and a lithium salt; alkaline earth metal salts such as a calcium salt, and a magnesium salt; metal salts such as an aluminum salt, an iron salt, a zinc salt, a copper salt, a nickel salt, and a cobalt salt; an ammonium salt; organic amine salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylammonium salt, and a tris(hydroxymethyl)aminomethane salt; hydrohalide salts such as a hydrofluoride salt, a hydrochloride salt, a hydrobromide salt, and a hydroiodide salt; inorganic acid salts such as a nitrate salt, a perchlorate salt, a sulfate salt, and a phosphate salt; lower alkanesulfonate salts such as a methanesulfonate salt, a trifluoromethanesulfonate salt, and an ethanesulfonate salt; arylsulfonate salts such as a benzenesulfonate salt, and p-toluenesulfonate salt; organic acid salts such as an acetate salt, a malate salt, a fumarate salt, a succinate salt, a citrate salt, a tartrate salt, an oxalate salt, and a maleate salt; and amino acid salts such as a glycine salt, a lysine salt, an arginine salt, an ornithine salt, a glutamate salt, an aspartate salt. These salts can be produced by a known method. Alternatively the antisense oligomer described herein may be in the form of a hydrate thereof.

The antisense oligomer described herein may have any of the groups represented in the following chemical formulae (1) to (3) at its 5'-end. Preferably, it is either group (1) or (2).

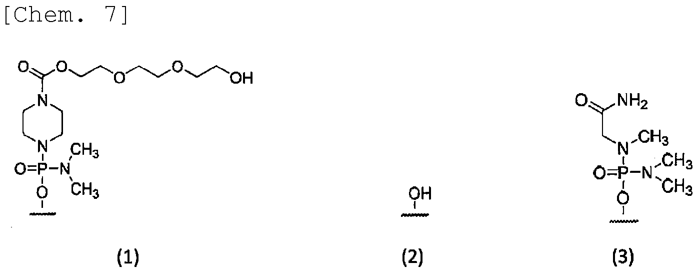

Hereinafter, the groups represented by (1), (2), and (3) above are referred to as "group (1)", "group (2)", and "group (3)", respectively.

The base sequence of the antisense oligomer described herein is not limited, and for example, the antisense oligomer may comprise four consecutive purine bases in its base sequence. Further, at least two of the four consecutive purine bases may be guanine.

In one embodiment, the antisense oligomer described herein comprises or consists of (i) a base sequence selected from the group consisting of SEQ ID NO: 1 to 12, (ii) a base sequence having a sequence identity of 80% or more, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more to the base sequence selected from the group consisting of SEQ ID NO: 1 to 12, or (iii) a basic sequence wherein one or several bases are added, deleted or substituted in the base sequence selected from the group consisting of SEQ ID NO: 1 to 12.

The term "identity" of the base sequences as used herein means a proportion of matched bases when two base sequences are aligned with each other. The sequence identity can be determined by using FASTA (Science 227 (4693): 1435-1441, (1985)) or the algorithm of Karlin and Altschul, BLAST (Basic Local Alignment Search Tool) (Proc. Natl. Acad. Sci. USA 872264-2268, 1990; Proc Natl Acad Sci USA 90: 5873, 1993). Programs based on the algorithm of BLAST and referred to as blastn, blastx, tblastn, and tblastx have been developed (Altschul SF, et al: J Mol Biol 215: 403, 1990). When blastn is used for base sequence analysis, parameters are set to, for example, score = 100, and wordlength = 12. When BLAST and Gapped BLAST programs are used, default parameters for each program are used.

As used herein, the term "several" in the base sequence wherein one or several bases are added, deleted, or substituted means 2, 3, 4, 5, 6, 7, 8, 9, or 10.

In one embodiment, the antisense oligomer described herein is one that targets a sequence raging from positions 115937 to 115981 of a genome sequence (SEQ ID NO: 13) of the insertion mutant of the fukutin gene wherein an SVA retrotransposon sequence (GenBank ACCESSION: AB185332) is inserted into the genome sequence of the fukutin gene (GenBank ACCESSION: AB038490) or one that does not target the sequence of the above range. Examples of the antisense oligomer that targets a sequence ranging from positions 115937 to 115981 of SEQ ID NO: 13 include antisense oligomers comprising a base sequence selected from the group consisting of SEQ ID NO: 1 to 7. Examples of the antisense oligomer that does not target a sequence ranging from positions 115937 to 115981 of SEQ ID NO: 13 include antisense oligomers comprising a base sequence selected from the group consisting of SEQ ID NO: 8 to 12.

In one embodiment, the antisense oligomer described herein is a conjugate to which a functional peptide, for example, cell-permeable peptide (CPP) is attached. Known functional peptides or commercially-available functional peptides can be used herein. Examples of the functional peptides that can be used herein include an arginine-rich peptide disclosed in International Publication No. 2008/036127; a peptide targeting organs disclosed in International Publication No. 2009/005793, for example, RXR, or RBR; and a peptide comprising amino acid subunits disclosed in International Publication No. 2012/150960. The cell-permeable peptide (CPP) can pass through cell membranes of mammalian cells, and accordingly, it represents a short peptide sequence having 10 to about 30 amino acids capable of improving cell drug delivery (see, e.g., Hum Mol Genet. 2011 Aug 15; 20(16): 3151-3160; Pharmacology & Therapeutics 154 (2015) 78-86). Known CPPs or commercially-available CPPs can be used herein. Examples of the CPPs that can be used herein include, the CPPs listed in Pharmacology & Therapeutics 154 (2015) 78-86, p.80, Table 1, such as TAT(48-60), penetratin, polyarginine, Oct4, WT1-pTj, DPV3, transportan, MAP, VP22, Rep1, KW, KFGF, FGF12, integrin β3 peptide, C105Y, TP2; and CPPs listed in Japanese Translation of PCT International Application Publication No. 2017-500856 (International Publication No. 2015/089487), paragraph [0085], Table 1, such as DPV10/6, DPV15b, YM-3, Tat, LR11, C45D18, Lyp-1, Lyp-2, BMV GAG, hLF1-22, C45D18, LR20. The CPPs are commercially available from Funakoshi, Co., Ltd., for example. Commercially available CPPs such as TAT (Funakoshi, Co., Ltd.), penetratin (Funakoshi, Co., Ltd.), or known CPPs such as R8 can be used herein. Examples of preferable CPPs that can be used herein include hLIMK, TAT, penetratin, and R8 (see, e.g., International Publication Nos. 2016/187425, 2018/118662, 2018/118599, and 2018/118627, and EBioMedicine 45 (2019) 630-645). The CPP can be directly bound to the antisense oligomer described herein, or can be bound via a linker capable of binding the CPP to the antisense oligomer. Known linkers can be used herein. Examples of the linker include those described in Japanese Translation of PCT International Application Publication No. 2017-500856 (International Publication No. 2015/089487), International Publication Nos. 2015/089487, 2009-073809, 2013/075035, 2015/105083, 2014/179620, 2015/006740, and 2017/010575. Examples of preferable linkers that can be used herein include 4-maleimidobutyrate, a linker capable of binding to the functional peptide or antisense oligomer described herein via disulfide bond. The conjugate as used herein can be prepared by a method known to those skilled in the art.

The pharmaceutical composition described herein may be formulated by appropriately blending a pharmaceutically acceptable carrier or additive (and optionally the sugar of the present invention). The pharmaceutically acceptable carrier or additive and formulation for the pharmaceutical composition is the same as those described for the precipitation suppressing agent of the present invention except that the antisense nucleic acid is the active ingredient.

In one embodiment, the precipitation suppressing agent of the present invention is used and/or added to the pharmaceutical composition in an amount such that the concentration of the sugar in the pharmaceutical composition described herein is 0.5 mg/mL to 3000 mg/mL, for example, 2.6 mg/mL to 1694 mg/mL. In one embodiment, the precipitation suppressing agent of the present invention is used and/or added to the pharmaceutical composition in an amount such that the concentration of the sugar in the pharmaceutical composition is 0.5 mg/mL or more, 1 mg/mL or more, 1.5 mg/mL or more, 2 mg/mL or more, 2.5 mg/mL or more, 3 mg/mL or more, 5 mg/mL or more, 10 mg/mL or more, 20 mg/mL or more, 30 mg/mL or more, 40 mg/mL or more, 50 mg/mL or more, or 100 mg/mL or more. Also, the precipitation suppressing agent of the present invention is used and/or added to the pharmaceutical composition in an amount such that the concentration of the sugar in the pharmaceutical composition is 3000 mg/mL or less, 2500 mg/mL or less, 2500 mg/mL or less, 2000 mg/mL or less, 1500 mg/mL or less, or 1000 mg/mL or less.

In one embodiment, the concentration of the antisense oligomer in the pharmaceutical composition described herein is 0.05 mg/mL to 2000 mg/mL, for example, 0.3 mg/mL to 350 mg/mL. In one embodiment, the concentration of the antisense oligomer in the pharmaceutical composition described herein may be 0.05 mg/mL or more, 0.1 mg/mL or more, 0.2 mg/mL or more, 0.3 mg/mL or more, 0.4 mg/mL or more, 0.5 mg/mL or more, 1 mg/mL or more, 5 mg/mL or more, or 10 mg/mL or more, and may be 2000 mg/mL or less, 1500 mg/mL or less, 1000 mg/mL or less, 750 mg/mL or less, 500 mg/mL or less, 400 mg/mL or less, 350 mg/mL or less, 300 mg/mL or less, or 250 mg/mL or less.

In one embodiment, the present invention relates to a precipitation suppressing agent for a pharmaceutical composition comprising an antisense oligomer, wherein the precipitation suppressing agent comprises a sugar and is used in an amount such that the weight ratio of the sugar is 0.1 to 100, for example, 1 to 53 per 1 of the antisense oligomer. The pharmaceutical composition comprising an antisense oligomer and the sugar are as described above. In one embodiment, the precipitation suppressing agent of the present invention is used in an amount such that the weight ratio of the sugar is 0.05 or more, 0.1 or more, 0.15 or more, 0.2 or more, 0.3 or more, 0.4 or more, 0.5 or more, 0.6 or more, 0.7 or more, 0.8 or more, 0.9 or more, 1 or more, 1.2 or more, 2 or more, or 5 or more per 1 of the antisense oligomer. In one embodiment, the precipitation suppressing agent of the present invention is used in an amount such that the weight ratio of the sugar is 100 or less, 53 or less, 40 or less, 30 or less, 25 or less, 20 or less, 15 or less, or 10 or less per 1 of the antisense oligomer.

In one embodiment, the precipitation suppressing agent of the present invention is comprised in the pharmaceutical composition described herein and administered therewith. For example, the antisense oligomer described herein is optionally lyophilized with a carrier such as glucose, and then dissolved in a solvent such as water for injection to prepare a pharmaceutical composition. The resulting pharmaceutical composition may be mixed with the precipitation suppressing agent described herein, and the amount of the mixture may then be optionally adjusted with a solvent to administer to a subject. Alternatively, for example, the antisense oligomer described herein is lyophilized with the precipitation suppressing agent of the present invention, and then dissolved in a solvent such as water for injection to prepare a pharmaceutical composition. The amount thereof may then be optionally adjusted with a solvent to administer to a subject.

In another embodiment, the precipitation suppressing agent of the present invention is not comprised in the pharmaceutical composition described herein, and is administered separately from (simultaneously or sequentially with) the pharmaceutical composition described herein. For example, a pharmaceutical composition obtained by dissolving a lyophilized agent of the antisense oligomer described herein in a solvent such as water for injection may be administered to a subject separately from the precipitation suppressing agent described herein. As used herein, administering the precipitation suppressing agent and the pharmaceutical composition "simultaneously" means administering the precipitation suppressing agent and the pharmaceutical composition at the same time point. As used herein, administering the precipitation suppressing agent and the pharmaceutical composition "sequentially" means administering each of them at a different time point. Specifically, the pharmaceutical composition can be administered before or after the precipitation suppressing agent is administered, and in this case, a dosing interval between the precipitation suppressing agent and the pharmaceutical composition is not limited, and may be, for example, several minutes, several hours, or up to about a day.

As used herein, examples of the subject to whom the pharmaceutical composition and/or precipitation suppressing agent are administered include, but not limited to, mammals, such as primates such as humans, laboratory animals such as rats, mice, and Norway rats, and farm animals such as pigs, cattle, horses, and sheep, and preferred are humans.

The dose of the pharmaceutical composition and/or precipitation suppressing agent when administered can be adjusted in consideration of the types of the antisense oligomer comprised in the pharmaceutical composition and the sugar comprised in the precipitation suppressing agent, the dosage form of the pharmaceutical composition and precipitation suppressing agent, the condition of the subject such as age or body weight, the administration route, and the nature and symptoms of the disease. The amount of the antisense oligomer can be in a range of 0.1 mg to 10 g/day/person, for example, in a range of 1 mg to 1 g/day/person, for example, or in a range of 10 mg to 100 mg/day/person, for example, and the amount of the sugar can be in a range of 0.1 mg to 200 g/day/person, for example, in a range of 1 mg to 100 g/day/person for example, in a range of 10 mg to 50 g/day/person, for example, in a range of 100 mg to 50 g/day/person, for example, in a range of 100 mg to 40 g/day/person, for example, in a range of 100 mg to 30 g/day/person, for example, in a range of 100 mg to 20 g/day/person, for example, in a range of 1 g to 20 g/day/person, for example, in a range of 2 g to 20 g/day/person, for example, in a range of 1 g to 10 g/day/person for example, or in a range of 100 mg to 1 g/day/person, for example. The number and frequency of administration are not limited, but for example, the administration can be performed once or two to three times a day at the interval of one day or two to three days. Further, for example, the administration can be performed only once, or another administration may be performed a few days later for a total of two administrations.

In one embodiment, the present invention relates to a method for suppressing precipitation of an antisense oligomer in urine, in a subject to whom an antisense oligomer or a pharmaceutical composition comprising an antisense oligomer has been administered, or a method for producing the pharmaceutical composition, comprising adding a sugar in an amount such that the concentration of the sugar in the pharmaceutical composition is 0.5 mg/mL to 3000 mg/mL. In the present embodiment, the antisense oligomer, the pharmaceutical composition, the sugar, the concentration of the sugar in the pharmaceutical composition and the like are as described herein.

In one embodiment, the present invention relates to a method for suppressing precipitation of an antisense oligomer in urine, in a subject to whom an antisense oligomer or a pharmaceutical composition comprising an antisense oligomer has been administered, comprising administering a sugar or a precipitation suppressing agent to the subject, wherein the sugar is administered in an amount such that the weight ratio of the sugar is 0.1 to 100 per 1 of the antisense oligomer. In the present embodiment, the antisense oligomer, the pharmaceutical composition, the sugar, the precipitation suppressing agent, the weight ratio of the sugar per 1 of the antisense oligomer and the like are as described herein.

In one embodiment, the present invention relates to a pharmaceutical composition comprising an antisense oligomer, and comprising a precipitation suppressing agent comprising a sugar at the concentration of 0.5 mg/mL to 3000 mg/mL. In the present embodiment, the antisense oligomer, the pharmaceutical composition, the sugar, the concentration of the sugar in the pharmaceutical composition and the like are as described herein.

### Examples

### [Example 1: Production of Antisense Oligomer (PMO)]

The antisense oligomers (PMO Nos. 1 to 12 (SEQ ID NO: 1 to 12)) listed in Table 1 were synthesized according to the method described in International Publication No. WO2013/100190. The theoretical value and measured value measured by ESI-TOF-MS of the molecular weight of each antisense oligomer are also shown.

**[Table 1]**

| Table 1: Synthesized antisense oligomers (PMO No. 1 to 12) | | | | | |
|---|---|---|---|---|---|
| PMO No. | Sequence | 5'-end | Molecular weight | | SEQ ID NO. |
| | | | Theoretical value | Measured value | |
| 1 | GCTCGGCATCAGAGGGAGACCG | Group (2) | 7353.55 | 7353.93 | 1 |
| 2 | CTCGGCATCAGAGGGAGACC | Group (2) | 6643.31 | 6643.91 | 2 |
| 3 | CGGCATCAGAGGGAGAC | Group (2) | 5682.99 | 5682.27 | 3 |
| 4 | CCTTGGCTCGGCATCAGAGG | Group (2) | 6625.29 | 6625.31 | 4 |
| 5 | CATCAGAGGGAGACCGTGTAA | Group (2) | 7021.45 | 7021.88 | 5 |
| 6 | CTCGTGCATCAGAGGGAGACC | Group (2) | 6973.42 | 6974.13 | 6 |
| 7 | CTCGCATCAGAGGGAGACC | Group (2) | 6288.19 | 6287.87 | 7 |
| 8 | AGGAGGGCAGCGAACTCCTGGTTGT | Group (2) | 8383.90 | 8383.85 | 8 |
| 9 | GTGGTTGAGCCAGAAGCTGGGAATT | Group (2) | 8422.91 | 8422.85 | 9 |
| 10 | GTTGCCTCCGGTTCTGAAGGTGTTC | Group (1) | 8643.00 | 8642.94 | 10 |
| 11 | CAATGCCATCCTGGAGTTCCTG | Group (1) | 7580.66 | 7580.17 | 11 |
| 12 | CCTCCGGTTCTGAAGGTGTTC | Group (2) | 6921.39 | 6921.66 | 12 |

"Group (1)" and "group (2)" in the table above are as follows.

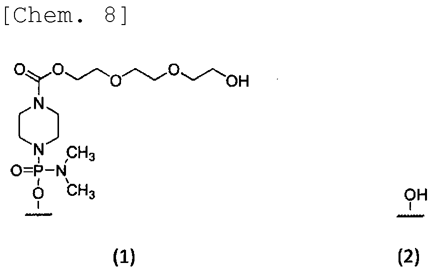

### [Example 2: Preliminary Study on Evaluation of Precipitation Suppressing Effects with Sugar]

For setting the test conditions such as the concentrations of sugar and antisense oligomers assumed to be in urine, D-mannitol, which is a monosaccharide and corresponds to a reduced form of D-mannose, and antisense oligomers were administered to cynomolgus monkeys, and the urinary concentration shortly after administration were estimated from the changes in plasma concentrations and the total body clearance.

20% D-mannitol was diluted to 50 mg/mL with physiological saline, the diluted solution thus obtained was administered at 10 mL/kg to 3 male and 3 female cynomolgus monkeys aged 1 to 2 year-old, and the plasma concentrations were then measured at 0 hours (immediately after administration), 0.25 hours, 1 hour, 4 hours, 8 hours, and 24 hours after administration (each of the values a, b, c, d, e, and f in Table 2 was an average value of 3 animals). From the values, the area under the plasma concentration-time curve (AUC_{0-24 hr}) up to 24 hours after administration and the total body clearance (CLₜₒₜ) were calculated (the values g and h in Table 2).
Further, the plasma concentrations at 0 hours and 0.25 hours after administration (the values a and b in Table 2) were averaged to obtain the average plasma concentration from 0 hours to 0.25 hours (the value i in Table 2), and the amount of D-mannitol excreted per body weight up to 0.25 hours (the value j in Table 2) was calculated by using CLₜₒₜ (the value h in Table 2). The amount of urine up to 0.25 hours after administration was assumed to be 3.91 mL, based on the fact that the daily amount of urine of cynomolgus monkeys (body weight: 5 kg) was 375 mL (B Clark, D A Smith Pharmacokinetics and toxicity testing. Crit Rev Toxicol. 1984; 12(4): 343-85.), and the urinary mannitol concentration at 0.25 hours after administration was then estimated (the value k in Table 2).

**[Table 2]**

| | Table 2: Estimation of urinary D-Mannitol concentration in cynomolgus monkeys | | |
|---|---|---|---|
| Male/female | | Male | Female |
| Amount administered | | 500 mg/kg | 500 mg/kg |
| | Plasma concentration 0 hr (a) | 2810 *µ*g/mL | 2950 *µ*g/mL |
| Plasma concentration 0. 25 hr (b) | | 1510 *µ*g/mL | 1490 *µ*g/mL |
| | Plasma concentration 1 hr (c) | 651 *µ*g/mL | 720 *µ*g/mL |
| | Plasma concentration 4 hr (d) | 89.2 *µ*g/mL | 90.3 *µ*g/mL |
| | Plasma concentration 8 hr (e) | 4.67 *µ*g/mL | 4.87 *µ*g/mL |
| Plasma concentration 24 hr (f) | | Below detection limit | Below detection limit |
| AUC_{0-24 hr} (g) | | 2680 *µ*g • hr/mL | 2830 *µ*g • hr/mL |
| CLₜₒₜ (h) | | 187 mL/hr/kg | 177 mL/hr/kg |
| Average plasma concentration from 0 hr to 0.25 hr (i) | | 2160 *µ*g/mL | 2220 *µ*g/mL |
| Amount excreted up to 0.25 hr (j) | | 100980 *µ*g/kg | 98235 *µ*g/kg |
| Estimated urinary concentration at 0.25 hr (k) | | 129 mg/mL | 126 mg/mL |

The estimated urinary concentration obtained as above was averaged, and the urinary concentration shortly after administration in a case of administering 500 mg/kg of D-mannitol was estimated to be 127.5 mg/mL. Since D-mannitol is rarely distributed in the body and rapidly excreted in urine without undergoing metabolism, it can be assumed that there is a linear relationship between the amount administered and the urinary concentration. Accordingly, by using the following formulas, the urinary concentration was converted into the amount of D-mannitol administered, and the amount administered was then converted into concentrations in the formulations at the time of administration (at the time of use) according to the body weight and the amount of dosing solution.

Amount of D-mannitol administered (mg/kg) = urinary concentration (mg/mL)/127.5 (mg/mL) × 500 (mg/kg) D-mannitol concentration in formulation (mg/mL) = amount of D-mannitol administered (mg/kg) × body weight (kg)/amount of dosing solution (mL)

At this time, the body weight and the amount of dosing solution were set to 3 to 80 kg and 50 to 200 mL, respectively.

Since the saccharides described herein such as sucrose do not undergo reabsorption in the renal tubules and exhibit biokinetics similar to those of D-mannitol (A W Winkler, J Parra. J Clin Invest. 1937 Nov; 16(6): 859-67.), it can be converted into concentrations in the formulations at the time of administration (at the time of use) by using the same conversion formulas. For example, an aqueous solution of sugar with an assumed urinary concentration of 135 mg/mL can be converted into the urinary concentration when administering 529.4 mg/kg of sugar, which is assumed that the formulation with the sugar concentration of 7.9 to 847.1 mg/mL is administered (used) .

Also, the antisense oligomer of PMO No. 12 (SEQ ID NO: 12) was dissolved to 1.6 mg/mL with physiological saline, the diluted solution thus obtained was administered at 10 mL/kg to 3 male cynomolgus monkeys aged 1 year-old, and the plasma concentrations were then measured at 0 hours (immediately after administration), 0.25 hours, 0.5 hours, 1 hour, 2 hours, 6 hours, and 24 hours after administration (each of the values a, b, c, d, e, f, and g in Table 3 was an average value of 3 animals). From the values, the area under the plasma concentration-time curve (AUC_{0-24 hr}) up to 24 hours after administration and the total body clearance (CLₜₒₜ) were calculated (the values h and i in Table 3). Further, the plasma concentrations at 0 hours and 0.25 hours after administration (the values a and b in Table 3) were averaged to obtain the average plasma concentration from 0 hours to 0.25 hours (the value j in Table 3), and the amount of antisense oligomer excreted per body weight up to 0.25 hours (the value k in Table 3) was calculated by using CLₜₒₜ (the value i in Table 3). The amount of urine up to 0.25 hours after administration was assumed to be 3.91 mL, based on the fact that the daily amount of urine of cynomolgus monkeys (body weight: 5 kg) was 375 mL, and the urinary antisense oligomer concentration at 0.25 hours after administration was then estimated (the value 1 in Table 3).

**[Table 3]**

| | Table 3: Estimation of urinary PMO No. 12 concentration in cynomolgus monkeys | | |
|---|---|---|---|
| Amount administered | | 10 mg/kg | 60 mg/kg |
| | Plasma concentration 0 hr (a) | 137.6 *µ*g/mL | 805.9 *µ*g/mL |
| Plasma concentration 0.25 hr (b) | | 30.95 *µ*g/mL | 154.7 *µ*g/mL |
| Plasma concentration 0. 5 hr (c) | | 19.06 *µ*g/mL | 100.9 *µ*g/mL |
| | Plasma concentration 1 hr (d) | 9.79 *µ*g/mL | 45.93 *µ*g/mL |
| | Plasma concentration 2 hr (e) | 4.328 *µ*g/mL | 18.46 *µ*g/mL |
| | Plasma concentration 6 hr (f) | Below detection limit | 1.045 *µ*g/mL |
| Plasma concentration 24 hr (g) | | Below detection limit | Below detection limit |
| AUC_{0-24 hr} (h) | | 50.25 *µ*g • hr/mL | 269.3 *µ*g • hr/mL |
| CLₜₒₜ (i) | | 199.0 mL/hr/kg | 222.8 mL/hr/kg |
| Average plasma concentration from 0 hr to 0.25 hr (j) | | 84.28 *µ*g/mL | 480.3 *µ*g/mL |
| Amount excreted up to 0.25 hr (k) | | 4193 *µ*g/kg | 26753 *µ*g/kg |
| Estimated urinary concentration at 0.25 hr (l) | | 5.362 mg/mL | 34.21 mg/mL |

Since it can be assumed from Table 3 that there is a linear relationship between the amount of antisense oligomer administered and the urinary concentration, by using the following formulas, the urinary concentration was converted into the amount of antisense oligomer administered, and the amount of antisense oligomer administered was then converted into concentrations in the formulations at the time of administration (at the time of use) according to the body weight and the amount of dosing solution.

Amount of antisense oligomer administered (mg/kg) = urinary concentration (mg/mL) / estimatied urinary concentration in cynomolgus monkey (mg/mL) × amount administered to cynomolgus monkey (mg/mL). Antisense oligmomer concentration in formulation (mg/mL) = amount of antisense oligomer administered (mg/kg) × body weight (kg)/amount of dosing solution (mL)

At this time, the value when 60 mg/kg of antisense oligomer was administered was used as the estimated urinary concentration in cynomolgus monkeys. Further, the body weight and the amount of dosing solution were set to 3 to 80 kg and 50 to 200 mL, respectively. For example, an aqueous solution of antisense oligomer with an assumed urinary concentration of 24.75 mg/mL can be converted into the urinary concentration when administering 43.41 mg/kg of antisense oligomer, which is assumed that the formulation with the antisense oligomer concentration of 0.7 to 69.5 mg/mL is administered (used) .

### [Example 3: Evaluation of Precipitation Suppressing Effects with Sucrose]

The amount of precipitation in the presence of ions assumed to be in urine and precipitation suppressing effects of sucrose were evaluated on the antisense oligomers of PMO Nos. 8, 9, 10, 11, among those listed in Table 1 to further verify their utility for pharmaceutical applications.

### Test Method

Each of the antisense oligomers was dissolved in water for injection or an aqueous solution of sucrose, and the mixture was then mixed with an aqueous solution comprising potassium chloride and sodium chloride assumed to be in urine. Evaluation conditions (solution compositions) are shown in Tables 4 to 11. The absorbance at 620 nm of the solution thus mixed was measured using a plate reader, Infinite F200 Pro (manufactured by Tecan Group Ltd.) under heating conditions of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of sucrose, and then the absorbance obtained under both the conditions was compared to each other. When the absorbance was increased, the precipitation was determined to be observed, and a time until the precipitation was observed and the increased value of the absorbance were evaluated. When the aqueous solution of sucrose was used as the medium, it was determined that the precipitation suppressing effect was presented in a case where the time until the precipitation was observed was longer or in a case where the increased value of the absorbance was smaller than the case of using the water for injection as the medium.

**[Table 4]**

| Table 4: Evaluation conditions of precipitation suppression by sucrose in PMO No. 8 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration. in formulation (converted value) (mg/mL) | Antisense oligomer concentration in formulation (converted value) (mg/mL) |
| 8 | 16.5 | 100 | 77 | 0 | 0 | 0.4∼46.3 |
| 8 | 16.5 | 100 | 77 | 135 | 7.9∼ 847.1 | 0. 4∼46.3 |
| 8 | 16.5 | 100 | 77 | 270 | 15.9∼ 1694.1 | 0. 4∼46.3 |

**[Table 5]**

| Table 5: Evaluation conditions of precipitation suppression by sucrose in PMO No. 8 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 8 | 14.667 | 200 | 154 | 0 | 0 | 0.4∼41.2 |
| 8 | 14.667 | 200 | 154 | 120 | 7.1∼ 752.9 | 0.4∼41.2 |

**[Table 6]**

| Table 6: Evaluation conditions of precipitation suppression by sucrose in PMO No. 8 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 8 | 16.5 | 100 | 77 | 0 | 0 | 0.4∼46.3 |
| 8 | 16.5 | 100 | 77 | 90 | 5.3∼ 564.7 | 0.4∼46.3 |
| 8 | 16.5 | 100 | 77 | 72 | 4.2∼ 451.8 | 0.4∼46.3 |
| 8 | 16.5 | 100 | 77 | 45 | 2.6∼ 282.4 | 0.4∼46.3 |

**[Table 7]**

| Table 7: Evaluation conditions of precipitation suppression by sucrose in PMO No. 9 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 9 | 12.375 | 100 | 77 | 0 | 0 | 0.3∼34.7 |
| 9 | 12.375 | 100 | 77 | 135 | 7.9∼ 847.1 | 0.3∼34.7 |
| 9 | 12.375 | 100 | 77 | 270 | 15.9∼ 1694.1 | 0.3∼34.7 |

**[Table 8]**

| Table 8: Evaluation conditions of precipitation suppression by sucrose in PMO No. 9 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 9 | 12.375 | 200 | 154 | 0 | 0 | 0.3∼34.7 |
| 9 | 12.375 | 200 | 154 | 120 | 7.1∼ 752.9 | 0.3∼34.7 |

**[Table 9]**

| Table 9: Evaluation conditions of precipitation suppression by sucrose in PMO No. 9 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 9 | 12.375 | 100 | 77 | 0 | 0 | 0.3∼34.7 |
| 9 | 12.375 | 100 | 77 | 90 | 5.3∼ 564.7 | 0.3∼34.7 |
| 9 | 12.375 | 100 | 77 | 72 | 4.2∼ 451.8 | 0.3∼34.7 |
| 9 | 12.375 | 100 | 77 | 45 | 2.6∼ 282.4 | 0.3∼34.7 |

**[Table 10]**

| Table 10: Evaluation conditions of precipitation suppression by sucrose in PMO No. 10 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 10 | 110 | 1500 | 385 | 0 | 0 | 2.9∼ 308.7 |
| 10 | 110 | 1500 | 385 | 75 | 4.4∼ 470.6 | 2.9∼ 308.7 |
| 10 | 110 | 1500 | 385 | 120 | 7.1∼ 752.9 | 2.9∼ 308.7 |

**[Table 11]**

| Table 11: Evaluation conditions of precipitation suppression by sucrose in PMO No. 11 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 11 | 110 | 1800 | 462 | 0 | 0 | 2.9∼ 308.7 |
| 11 | 110 | 1800 | 462 | 120 | 7.1∼ 752.9 | 2.9∼ 308.7 |
| 11 | 110 | 1800 | 462 | 60 | 3.5∼ 376.5 | 2.9∼ 308.7 |

### Test Results

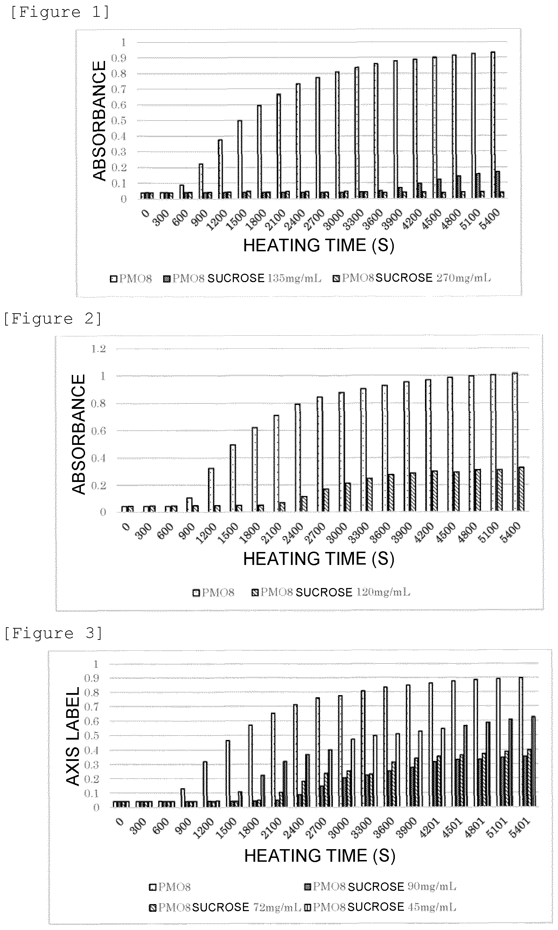

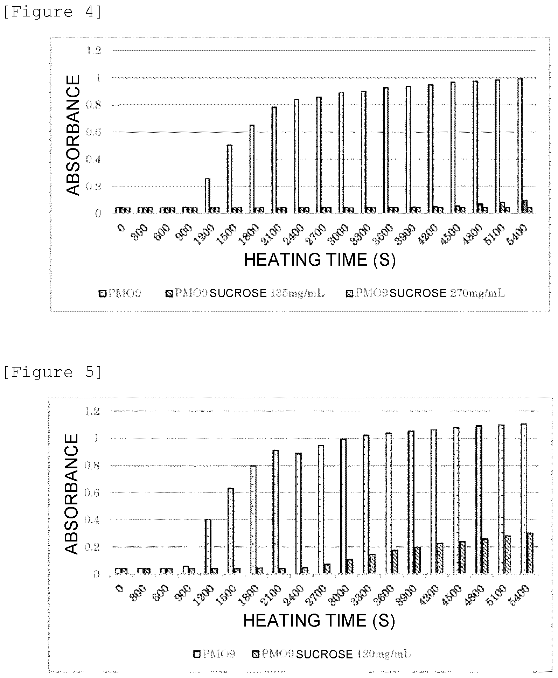

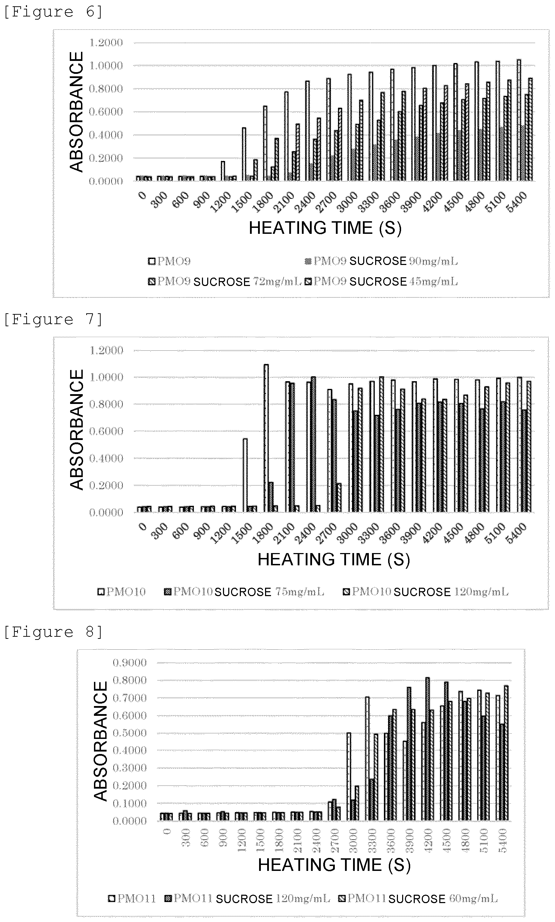

Since each of the antisense oligomers tested prolonged the time until the precipitation was observed and suppressed the increase of the absorbance in all the case of using the aqueous solution of sucrose as the medium, sucrose was indicated to suppress the precipitation of antisense oligomers. The respective results are shown in Figures 1 to 8.

The above results showed that sucrose suppresses the precipitation of the antisense oligomers in urine.

### [Example 4: Evaluation of Precipitation Suppressing Effects in Kidney with Sucrose]

Precipitation of the antisense oligomer of PMO No. 8 (SEQ ID NO: 8) among those listed in Table 1 from urine and precipitation suppression with sucrose in the kidney were evaluated to verify its further utility for pharmaceutical applications.

### (1) Evaluation Method

The antisense oligomer of PMO No. 8 was dissolved in water for injection comprising 0.9% sodium chloride, and a solution obtained by dissolving sucrose (manufactured by FUJIFILM Wako Pure Chemical Corporation) with physiological saline was added thereto to prepare a dosing solution comprising each of the antisense oligomer and sucrose at the concentrations shown in Tables 12 and 13.

The obtained dosing solutions were administered into the tail vein of 6-week-old C57BL/6J male mice (N=3) at the amount of dosing solution shown in Table 12 (10 mL/kg). Next day, some mice to which the antisense oligomer of PMO No. 8 had been administered were subjected to autopsy, and formalin-fixed paraffin-embedded samples of the respective kidneys were prepared. Sections obtained by slicing the samples were subjected to hematoxylin-eosin staining and a histopathological test of the sections was performed.

**[Table 12]**

| Table 12: Dosing solution of antisense oligomer (PMO No. 8) and amount of dosing solution | | | |
|---|---|---|---|
| Amount of dosing solution (mL/kg) | Concentration of PMO (mg/mL) | Concentration of Sucrose (mg/mL) | Weight ratio of Sucrose/PMO |
| - | - | - | - |
| 10 | 50 | 0 | 0 |
| 10 | 50 | 5 | 0.1 |
| 10 | 50 | 10 | 0.2 |
| 10 | 50 | 20 | 0.4 |
| 10 | 50 | 50 | 1 |
| 10 | 50 | 100 | 2 |

| | | | |
|---|---|---|---|
| -: No administration | | | |

### (2) Evaluation Results

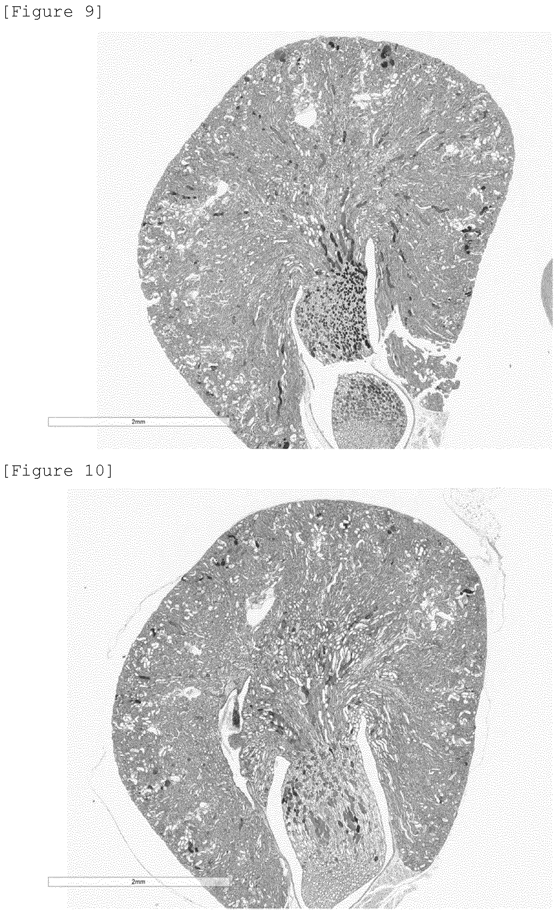

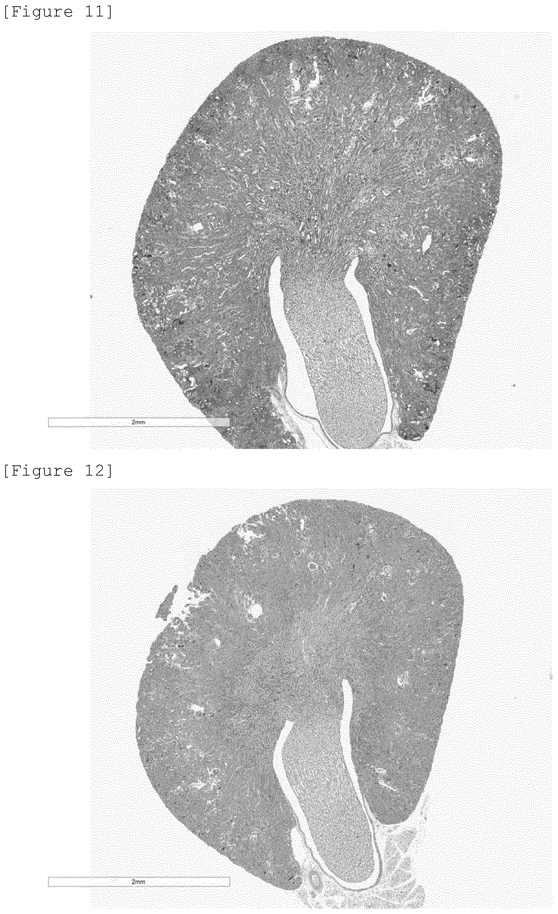

The histopathological test revealed that basophilic substances were observed in the lumens of renal tubules in the kidneys of the mice to which the control dosing solution comprising no sucrose has been administered. This change was thought to be caused by precipitation of nucleic acids. The amount of precipitates decreased according to the concentrations of sucrose, which confirmed the precipitation suppressing effects. The results are shown in Table 13 and Figures 9, 10, 11, and 12.

**[Table 13]**

| Table 13: Histopathological test of kidney when administering antisense oligomer (PMO No. 8) | | | | | | |
|---|---|---|---|---|---|---|
| Amount of dosing solution (mL/kg) | Concentration of PMO (mg/mL) | Concentration of sucrose (mg/mL) | Weight ratio of sucrose/PMO | Basophilic substances in renal tubules | Expansion of renal tubules | Renal tubular necrosis in inner stripe of outer medulla |
| - | - | - | - | - | - | - |
| 10 | 50 | 0 | 0 | 2+ (3/3) | 3+ (3/3) | ± (3/3) |
| 10 | 50 | 20 | 0.4 | 2+ (3/3) | 3+ (3/3) | 1+ (1/3) ± (2/3) |
| 10 | 50 | 50 | 1 | 1+ (3/3) | 2+ (1/3) 1+ (2/3) | ± (1/3) |
| 10 | 50 | 100 | 2 | ± (3/3) | - | - |

| | | | | | | |
|---|---|---|---|---|---|---|
| -: Not changed ±: The case where the change is very slight, and thus the organ/tissue is considered to function normally. 1+: The case where the change is definite but its distribution or degree is limited, and thus the organ/tissue is considered to be rarely affected functionally. 2+: The case where the change is definite and observed widely, and thus the organ/tissue is considered to be affected to some extent functionally. 3+: The case where the change is significant, and thus the organ/tissue is considered to be definitely affected functionally. | | | | | | |

### [Example 5: Evaluation of Precipitation Suppressing Effects in Kidney with Sucrose]

Precipitation of the antisense oligomer of PMO No. 11 (SEQ ID NO: 11) among those listed in Table 1 from urine and precipitation suppression with sucrose in the kidney were evaluated to verify its further utility for pharmaceutical applications.

### (1) Evaluation Method

The antisense oligomer of PMO No. 11 was dissolved in physiological saline to prepare a control dosing solution comprising the antisense oligomer at the concentration shown in Table 14 and comprising no sucrose.

Next, the antisense oligomer of PMO No. 11 and sucrose were dissolved in physiological saline to prepare dosing solutions comprising the antisense oligomer and sucrose at the concentrations shown in Table 14.

The obtained dosing solutions were administered into the tail vein of 6-week-old C57BL/6J male mice (N=5) at 20 mL/kg. Next day, the mice were subjected to autopsy, and formalin-fixed paraffin-embedded samples of the respective kidneys were prepared. Sections obtained by slicing the samples were subjected to hematoxylin-eosin staining and a histopathological test of the sections was performed. Compared to the mice to which the control dosing solution comprising no sucrose has been administered, it was determined that the precipitation was suppressed in a case where there was improvement in the findings on the histopathological test of the kidney of the mice to which the dosing solution comprising sucrose has been administered.

### (2) Evaluation Results

Since the antisense oligomer tested was confirmed that improvement in the findings was observed on the histopathological test of the kidney of the mice to which the dosing solution comprising sucrose has been administered, sucrose was confirmed to suppress precipitation. The results are shown in Table 14.

**[Table 14]**

| Table 14: Findings on histopathological test of kidney when administering antisense oligomer (PMO No. 11) | | | | | |
|---|---|---|---|---|---|
| Amount of dosing solution (mL/kg) | Concentration of PMO (mg/mL) | Concentration of sucrose (mg/mL) | Weight ratio of sucrose/PMO | Renal tubular necrosis in outer stripe of outer medulla | Hemorrhage/ congestion in inner stripe of outer medulla |
| 20' | 0 | 0 | 0 | - | - |
| 20 | 130 | 0 | 0 | ± (3/5) | ± (2/5) |
| 20 | 130 | 200 | 1. 538 | ± (1/5) | - |

| | | | | | |
|---|---|---|---|---|---|
| *: Physiological saline administered -: Not changed ±: The case where the change is very slight, and thus the organ/tissue is considered to function normally. | | | | | |

### [Example 6: Evaluation of Precipitation Suppressing Effects with Sucrose]

The amount of precipitation in the presence of ions assumed to be in urine and precipitation suppressing effects of sucrose were evaluated on the antisense oligomers of PMO Nos. 1, 2, and 7 among those listed in Table 1 to further verify their utility for pharmaceutical applications.

### Test Method

Each of the antisense oligomers was dissolved in water for injection or an aqueous solution of sucrose, and the mixture was then mixed with an aqueous solution comprising potassium chloride and sodium chloride assumed to be in urine. Evaluation conditions (solution compositions) are shown in Tables 4 to 11 and 15 to 17. The absorbance at 620 nm of the solution thus mixed was measured using a plate reader, Infinite F200 Pro (manufactured by Tecan Group Ltd.) under heating conditions of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of sucrose, and then the absorbance obtained under both the conditions was compared to each other. When the absorbance was increased, precipitation was determined to be observed, and a time until the precipitation was observed and the increased value of the absorbance were evaluated. When the aqueous solution of sucrose was used as the medium, it was determined that the precipitation suppressing effect was presented in a case where the time until the precipitation was observed was longer or in a case where the increased value of the absorbance was smaller than the case of using the water for injection as the medium.

**[Table 15]**

| Table 15: Evaluation conditions of precipitation suppression by sucrose in PMO No. 1 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 1 | 24. 75 | 100 | 77 | 0 | 0 | 0.7∼69.5 |
| 1 | 24. 75 | 100 | 77 | 135 | 7.9∼ 847.1 | 0.7∼69.5 |
| 1 | 24. 75 | 100 | 77 | 45 | 2.6∼ 282.4 | 0.7∼69.5 |

**[Table 16]**

| Table 16: Evaluation conditions of precipitation suppression by sucrose in PMO No. 2 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 2 | 49.5 | 100 | 77 | 0 | 0 | 1.3∼ 138.9 |
| 2 | 49.5 | 100 | 77 | 135 | 7.9∼ 847.1 | 1.3∼ 138.9 |
| 2 | 49.5 | 100 | 77 | 45 | 2.6∼ 282.4 | 1.3∼ 138.9 |

**[Table 17]**

| Table 17: Evaluation conditions of precipitation suppression by sucrose in PMO No. 7 | | | | | | |
|---|---|---|---|---|---|---|
| PMO No. | PMO ( mg/mL) | KCl ( mmol/mL) | NaCl ( mmol/mL) | Sucrose (mg/mL) | Sucrose concentration in formulation (converted value) (mg/mL) | Antisense oligomer concentration in formulation (converted value) (mg/mL) |
| 7 | 264 | 400 | 154 | 0 | 0 | 6.9∼ 740.8 |
| 7 | 264 | 400 | 154 | 120 | 7.1∼ 752.9 | 6.9∼ 740.8 |
| 7 | 264 | 400 | 154 | 80 | 4.7∼ 502.0 | 6.9∼ 740.8 |

### Test Results

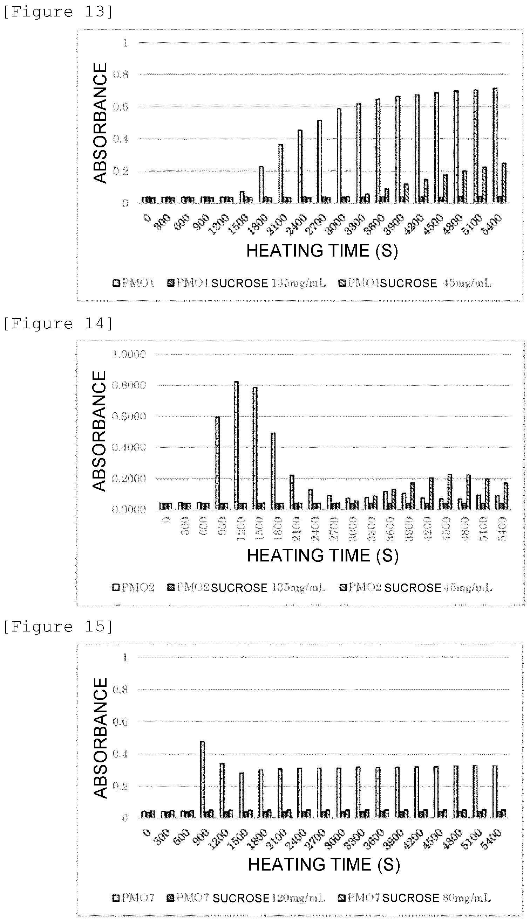

Since each of the antisense oligomers tested prolonged the time until the precipitation was observed and suppressed the increase of the absorbance in all the case of using the aqueous solution of sucrose as the medium, sucrose was indicated to suppress the precipitation of antisense oligomers. The respective results are shown in Figures 13 to 15.

The above results showed that sucrose suppresses the precipitation of the antisense oligomers in urine.

### [Example 7: Evaluation of Precipitation Suppressing Effects with Trehalose]

The amount of precipitation in the presence of ions assumed to be in urine and precipitation suppressing effects of trehalose were evaluated on the antisense oligomers of PMO Nos. 1, 2, 7, and 8 among those listed in Table 1 to further verify their utility for pharmaceutical applications.

### Test Method

Each of the antisense oligomers was dissolved in water for injection or an aqueous solution of trehalose, and the mixture was then mixed with an aqueous solution comprising potassium chloride and sodium chloride assumed to be in urine. Evaluation conditions (solution compositions) are shown in Tables 18 to 21. The absorbance at 620 nm of the solution thus mixed was measured using a plate reader, Infinite F200 Pro (manufactured by Tecan Group Ltd.) under heating conditions of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of trehalose, and then the absorbance obtained under both the conditions was compared to each other. When the absorbance was increased, precipitation was determined to be observed, and a time until the precipitation was observed and the increased value of the absorbance were evaluated. When the aqueous solution of trehalose was used as the medium, it was determined that the precipitation suppressing effect was presented in a case where the time until the precipitation was observed was longer or in a case where the increased value of the absorbance was smaller than the case of using the water for injection as the medium.

**[Table 18]**

| Table 18: Evaluation conditions of precipitation suppression by trehalose in PMO No. 1 | | | | | | |
|---|---|---|---|---|---|---|
| PMO No. | PMO ( mg/mL) | KCl ( mmol/mL) | NaCl ( mmol/mL) | Trehalose (mg/mL) | Trehalose concentration in formulation (converted value) (mg/mL) | Antisense oligomer concentration in formulation (converted value) (mg/mL) |
| 1 | 24.75 | 100 | 77 | 0 | 0 | 0.7∼69.5 |
| 1 | 24.75 | 100 | 77 | 45 | 2.6 282.4 | 0.7∼69. 5 |
| 1 | 24.75 | 100 | 77 | 90 | 5.3∼ 564.7 | 0.7∼69.5 |
| 1 | 24.75 | 100 | 77 | 135 | 7.9∼ 847.1 | 0.7∼69.5 |
| 1 | 24.75 | 100 | 77 | 180 | 10.6∼ 1129.4 | 0.7∼69.5 |

**[Table 19]**

| Table 19: Evaluation conditions of precipitation suppression by trehalose in PMO No. 2 | | | | | | |
|---|---|---|---|---|---|---|
| PMO No. | PMO ( mg/mL) | KCl ( mmol/mL) | NaCl ( mmol/mL) | Trehalose (mg/mL) | Trehalose concentration in formulation (converted value) (mg/mL) | Antisense oligomer concentration in formulation (converted value) (mg/mL) |
| 2 | 49.5 | 100 | 77 | 0 | 0 | 1.3∼ 138.9 |
| 2 | 49.5 | 100 | 77 | 45 | 2.6∼ 282.4 | 1.3∼ 138.9 |

**[Table 20]**

| Table 20: Evaluation conditions of precipitation suppression by trehalose in PMO No. 7 | | | | | | |
|---|---|---|---|---|---|---|
| PMO No. | PMO ( mg/mL) | KCl ( mmol/mL) | NaCl ( mmol/mL) | Trehalose (mg/mL) | Trehalose concentration in formulation (converted value) (mg/mL) | Antisense oligomer concentration in formulation (converted value) (mg/mL) |
| 7 | 264 | 400 | 154 | 0 | 0 | 6.9∼ 740. 8 |
| 7 | 264 | 400 | 154 | 80 | 4.7∼ 502. 0 | 6.9∼ 740.8 |

**[Table 21]**

| Table 21: Evaluation conditions of precipitation suppression by trehalose in PMO No. 8 | | | | | | |
|---|---|---|---|---|---|---|
| PMO No. | PMO ( mg/mL) | KCl ( mmol/mL) | NaCl ( mmol/mL) | Trehalose (mg/mL) | Trehalose concentration in formulation (converted value) (mg/mL) | Antisense oligomer concentration in formulation (converted value) (mg/mL) |
| 8 | 16. 5 | 100 | 77 | 0 | 0 | 0.4∼46.3 |
| 8 | 16.5 | 100 | 77 | 45 | 2.6∼ 282.4 | 0.4∼46.3 |
| 8 | 16. 5 | 100 | 77 | 90 | 5.3∼ 564.7 | 0.4∼46.3 |
| 8 | 16. 5 | 100 | 77 | 135 | 7.9∼ 847.1 | 0.4∼46.3 |

### Test Results

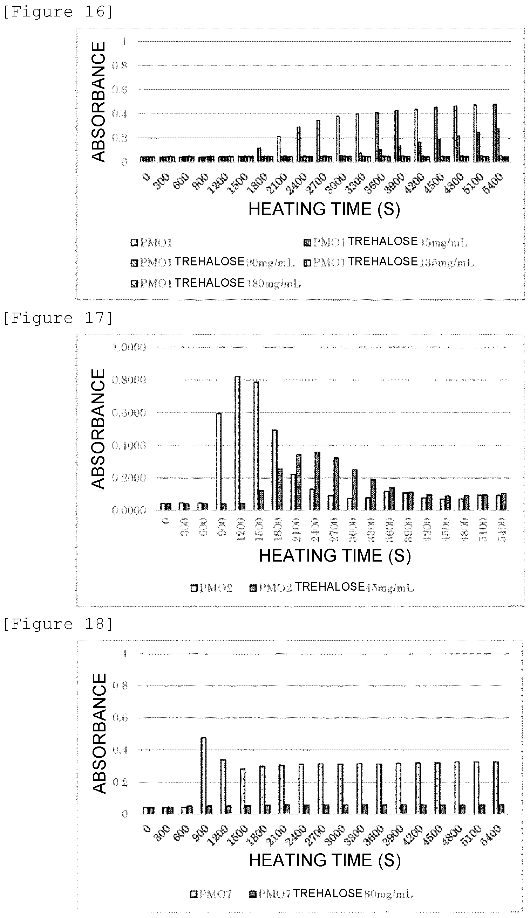

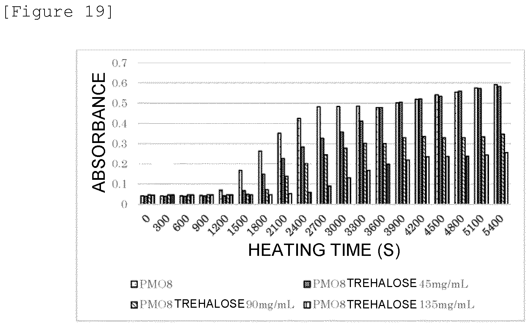

Since each of the antisense oligomers tested prolonged the time until the precipitation was observed and suppressed the increase of the absorbance in all the case of using the aqueous solution of trehalose as the medium, trehalose was indicated to suppress the precipitation of antisense oligomers. The respective results are shown in Figures 16 to 19.

The above results showed that trehalose suppresses the precipitation of the antisense oligomers in urine.

## Claims

1. A precipitation suppressing agent for an antisense oligomer in urine of a subject to whom a pharmaceutical composition comprising an antisense oligomer has been administered, wherein the precipitation suppressing agent comprises a sugar other than glucose and is used in an amount such that a concentration of the sugar in the pharmaceutical composition is 0.5 mg/mL to 3000 mg/mL.

2. The precipitation suppressing agent according to claim 1, wherein the precipitation suppressing agent is used in an amount such that the concentration of the sugar in the pharmaceutical composition is 2.6 mg/mL to 1694.1 mg/mL.

3. The precipitation suppressing agent according to claim 1 or 2, wherein a concentration of the antisense oligomer in the pharmaceutical composition is 0.05 mg/mL to 2000 mg/mL.

4. A precipitation suppressing agent for an antisense oligomer in urine of a subject to whom a pharmaceutical composition comprising an antisense oligomer has been administered, wherein the precipitation suppressing agent comprises a sugar other than glucose and is used in an amount such that a weight ratio of the sugar is 0.1 to 100 per 1 of the antisense oligomer.

5. The precipitation suppressing agent according to claim 4, wherein the precipitation suppressing agent is used in an amount such that the weight ratio of the sugar is 1 to 53 per 1 of the antisense oligomer.

6. The precipitation suppressing agent according to any one of claims 1 to 5, wherein the sugar is a disaccharide.

7. The precipitation suppressing agent according to any one of claims 1 to 6, wherein the sugar is sucrose.

8. The precipitation suppressing agent according to any one of claims 1 to 6, wherein the sugar is trehalose.

9. The precipitation suppressing agent according to any one of claims 1 to 8, wherein the antisense oligomer is a morpholino oligomer.

10. The precipitation suppressing agent according to any one of claims 1 to 9, wherein the antisense oligomer is a phosphorodiamidate morpholino oligomer.

11. The precipitation suppressing agent according to any one of claims 1 to 10, wherein the 5'-end of the antisense oligomer is any group represented by the following chemical formulae (1) and (2):

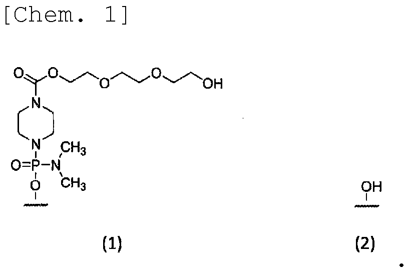

12. The precipitation suppressing agent according to any one of claims 1 to 11, wherein the antisense oligomer comprises four consecutive purine bases in its base sequence.

13. The precipitation suppressing agent according to claim 12, wherein at least two of the four consecutive purine bases are guanine.

14. The precipitation suppressing agent according to any one of claims 1 to 13, wherein the antisense oligomer comprises a base sequence selected from the group consisting of SEQ ID NOs: 1 to 12.

15. A method for suppressing precipitation of an antisense oligomer in urine, in a subject to whom a pharmaceutical composition comprising an antisense oligomer has been administered, comprising adding a sugar other than glucose in an amount such that a concentration of the sugar in the pharmaceutical composition is 0.5 mg/mL to 3000 mg/mL.

16. A method for suppressing precipitation of an antisense oligomer in urine, in a subject to whom an antisense oligomer has been administered, comprising administering a sugar other than glucose to the subject, wherein the sugar is administered in an amount such that a weight ratio of the sugar is 0.1 to 100 per 1 of the antisense oligomer.

17. A pharmaceutical composition comprising an antisense oligomer, wherein the pharmaceutical composition comprises a precipitation suppressing agent at a concentration of 0.5 mg/mL to 3000 mg/mL for an antisense oligomer in urine of a subject to whom the pharmaceutical composition has been administered, the precipitation suppressing agent comprising a sugar other than glucose.
